# EUROPEAN PATENT APPLICATION

(11) **EP 2 612 849 A1**
(43) Date of publication of application: **10.07.2013**
(21) Application number: 12150256.1
(22) Date of filing: 05.01.2012
(51) Int. Cl.: C07C 407/00, C07C 409/10

(54) **Improved cumene hydroperoxide concentration process**

(71) Applicant: Borealis AG, 1220 Vienna (AT)
(72) Inventor: PUROLA, Veli-Matti, FI-06650 Porvoo (FI)
(74) Representative: Campbell, Neil Boyd

(57) **Abstract**

A process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least the first of said concentration columns is provided with a fresh cumene feed.

## Description

This invention relates to the manufacture of phenol from cumene and in particular to the cumene hydroperoxide concentration step in the early stages of the phenol production process. The invention specifically relates to the use of random or structured packing in the distillation columns used during the concentration process and/or to the use of a dedicated fresh cumene feed to at least the first of the distillation columns of the concentration process.

### Background

Phenol is commonly manufactured from cumene, wherein cumene is oxidized to cumene hydroperoxide (CHP) and the resulting oxidation product mixture is concentrated and subjected to a cleavage reaction to form acetone and phenol. The oxidation reaction generally follows the following scheme:

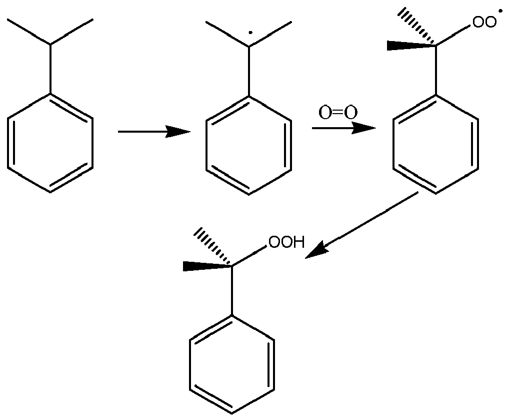

All cumene based phenol production processes generally have the following steps therefore:
1. Oxidation of cumene to CHP;
2. Concentration of the oxidation products to enhance CHP content within the reaction medium;
3. Acidic cleavage of the CHP (and impurities therein) to form phenol, acetone and impurities or byproducts (e.g. AMS);
4. Neutralisation of the acid;
5. Separation and purification of reaction products.

This invention relates to stage 2 above. Before the CHP is subjected to a cleavage reaction therefore, the concentration of CHP in the reaction mixture from oxidation must be enhanced, i.e. concentrated. This is called the concentration step herein. This is typically achieved in two or three stages to increase the CHP concentration in the reaction mixture from around 25-35 wt% up to 75-82 wt%. The main aim of this step is to separate cumene and CHP and remove unreacted cumene to be recycled to the start of the oxidation process.

The concentration step typically involves a first flash step followed by one or more distillation steps. In WO2010/069586, the concentration of CHP is discussed in which a first step involves a flash step or distillation with reflux and a second step involves distillation with reflux. The final stage carries the smallest load since the majority of the cumene is removed in the first stage(s) of the process. Unreacted cumene can be condensed, washed and recycled to the oxidation step.

A similar disclosure of the cumene concentration step can be found in WO2010/078934.

Other technologies than conventional distillation have also been proposed for CHP purification. In US4654124, purification of the CHP is achieved using wiped film evaporators. The process involves a series of pressure and temperature controlled evaporations. It is alleged that the pressure and temperature control prevents degradation of the CHP during concentration.

There remain however, problems during the cumene hydroperoxide concentration step which the skilled man must prevent. Cumene hydroperoxide loss during the concentration step is typically about 0.1 to 1.4 %, depending on the impurities carried with the oxidation reaction mixture and the design of concentration section. This is undesirable.

Thermal decomposition of CHP to unwanted side products like methanol, dimethylphenolcarbinol and acetophenone increases if, for example alkaline impurities are present. A detailed description of the effect of impurities on CHP thermal decomposition is presented elsewhere (Ind. Eng. Chem. Res. Wang et al; Thermal runaway Hazards of Cumene Hydroperoxide with contaminants).

Acidic impurities like strong organic acids also increase decomposition of CHP to unwanted products. The skilled artisan obviously wants to minimise formation of these side materials and CHP loss.

Moreover, if the concentration is not carefully controlled, runaway reaction can occur. Factors affecting to possible runaway during CHP concentration include the residence time within the concentration step, the presence of impurities such as salts and carbonates, organic acids as well as the operating temperature.

It is generally the case that if residence times within the concentration step are minimised and the presence of salts minimised then safety and success of the cumene to concentrated CHP process can be improved.

The present inventors have devised further improvements to the concentration step in a phenol production process. By adopting one or more of the techniques mentioned below, significant improvements to the phenol production process and in particular the concentration process can be achieved. In particular, the yield of CHP from cumene can be improved and the amount of impurities can be reduced. By adopting the strategies below, residence time within the concentration step can also be reduced thus minimising the chance of CHP degradation and runaway.

The present inventors have now devised an improved process for the concentration of the CHP to levels required for use in the cleavage reaction to give acetone and phenol.

### Summary of Invention

Viewed from one aspect the invention provides a process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
   wherein at least the first of said concentration columns is provided with a fresh cumene feed, preferably to the top of the column.

Viewed from one aspect the invention provides a process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least one of said concentration columns comprises random or structured packing.

Viewed from one aspect the invention provides a process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least one of said concentration columns comprises random or structured packing; and
wherein at least the first of said concentration columns is provided with a fresh cumene feed, preferably to the top of the column.

Viewed from another aspect the invention provides a process for the conversion of cumene to cumene hydroperoxide and the subsequent concentration thereof comprising:
(I) oxidising cumene in the presence of an oxygen containing gas in at least one oxidation reactor, preferably at least 3 oxidation reactors connected in series, so as to form a reaction mixture containing cumene and cumene hydroperoxide;
(II) transferring the reaction mixture of stage (I) to the first of at least two concentration columns arranged in series;
(III) distilling the reaction mixture in said concentration columns to increase the percentage of cumene hydroperoxide within the reaction mixture and removing cumene from the reaction mixture in each concentration column via an overhead stream;
wherein at least the first of said concentration columns is provided with a fresh cumene, preferably to the top of the column; and/or
wherein at least one of said concentration columns comprises random or structured packing.

Viewed from another aspect the invention provides an apparatus for use in a process as hereinbefore defined comprising:
a cumene source (1) arranged to provide cumene to a first oxidation reactor (2), said first oxidation reactor being the first in a series of at least three oxidation reactors connected in series;
at least two concentration columns (3A-3C) connected in series arranged to receive reaction mixture from the last in said series of oxidation reactors;
said concentration columns each having an overhead conduit arranged to remove cumene from said columns for recycling via a caustic (4) and then water (5) washing means to said cumene source (1);
said cumene source being arranged to provide fresh cumene via a conduit (14) to at least the first of said concentration columns.
One of more of said concentration columns may comprise random or structured packing.

Viewed from another aspect the invention provides the use of the apparatus as hereinbefore defined in the concentration of cumene hydroperoxide

### Detailed Description of the Invention

### Oxidation step

The first step of the cumene process involves the conversion of cumene to CHP. That can be achieved using conventional oxidation methods such as discussed in WO2010/069586. Any oxidation technology can therefore be used in this step of the process, preferably such as described below.

Whilst it is in theory possible to use a single oxidation reactor, it is preferred if there are 3-8, more preferably 3-6 reactors.

The oxidation step takes place in the presence of an oxygen containing gas such as air. The oxygen containing gas feed is preferably air. However, a more concentrated oxygen gas may be used. The oxygen content of the oxygen containing gas may be up to 100%, preferably about 22-80% oxygen. The other components of the oxygen containing gas feed should be inert gases, typically nitrogen. In a preferred embodiment, air is used without modification (i.e. without an oxygen enrichment) other than standard cleaning/purification procedures.

The process of the invention comprises conducting cumene to at least the first reactor in a series of oxidation reactors. Typically the cumene feed is quite pure and essentially contains cumene and minor amounts of impurities. Preferably therefore, this feed is at least 95 wt% cumene, preferably at least 99 wt% cumene. It may contain cumene recycled from the concentration step as explained in further detail below. In this regard, the treatment of the recycled cumene discussed below ensures that any cumene reused in the oxidation process contains very low amounts of carbonate impurities.

Preferably, the cumene is free of such impurities, i.e. no carbonates are detectable. Such impurities have been found to reduce yields during the concentration step as they foul the surface of reboilers used to drive distillation.

The cumene feed is preferably dry. By dry is meant less than 0.5 wt% water, preferably less than 0.1 wt% water. In order to maximise the dryness of the cumene, the tank from which cumene is supplied to the first oxidation reactor can be provided with a water drain to remove any water present. Cumene and water are immiscible so water can be drained by phase separation. As the cumene in the tank will contain cumene recycled from the concentration process which has been washed, there may well be carry over of water from the washing step into the cumene tank. This can be readily removed using a water drain.

The cumene feed is preferably a liquid feed. Whilst this fresh cumene feed can be fed to more than one reactor in a series of oxidation reactors, it is especially preferred if fresh cumene (i.e. cumene not part of the oxidation mixture) is fed to the first reactor in the series only. For the avoidance of doubt the term cumene feed is used herein to refer to a feed of cumene deriving from a substantially pure cumene source. Where cumene might form part of a reaction mixture (e.g. part of the reaction mixture transferred from oxidation to concentration steps), we do not regard that as a cumene feed.

Along with cumene, an oxygen containing gas is also feed to at least the first reactor in the series of oxidation reactors to thereby form an oxidation mixture. The cumene feed and oxygen containing gas feed are preferably fed separately although conceivably these could be fed together. Once the oxidation mixture is formed in the first reactor, CHP forms. That part of the oxidation mixture which is displaced by the liquid being fed into a reactor is then preferably conducted from the first reactor to at least one subsequent reactor, preferably the next one in the series. It is within the scope of the invention however for the oxidation mixture to be split and fed to one or more subsequent reactors. It is also within the scope of the invention for some transfers to occur in series and some transfers to be split and fed to different reactors.

Thus reactors can be arranged in parallel or in series or a mixture thereof. Other than in the last reactor, oxidation mixture from a reactor should be transferred to at least one downstream reactor in the series. Oxidation mixture from each reactor is preferably transferred to at least the next reactor downstream thereof in the series. The reactors in the process of the invention are preferably connected only in series so that the oxidation mixture passes from one reactor to the next one in the series. It will be appreciated that the process of the invention will run continuously so there will always be new feed material entering and reacted material leaving the reactor.

Thus, to each subsequent reactor in the series (i.e. not the first), material is preferably transferred from the previous reactor. Thus, a liquid feed comprising unreacted cumene, its oxidation product (CHP) and any impurities is preferably fed from reactor to reactor in the series as the oxidation mixture. Thus, whilst cumene is formally transferred to each reactor in the series, it is preferably only the first reactor that has a dedicated and preferably essentially pure cumene feed. To all other reactors, any cumene is preferably added only as an unreacted part of the transferring oxidation mixture.

It will be appreciated that the amount of cumene in the oxidation mixture will reduce as the oxidation mixture passes from reactor to reactor as more cumene is converted to CHP and more removed from the top gases. As the reaction progresses therefore, each transfer mixture preferably contains less cumene and more CHP than the previous transfer mixture.

An oxygen containing gas is also preferably fed, preferably to the bottom section, of each oxidation reactor, in the series, thereby maintaining an oxidation mixture in each reactor. Unlike cumene therefore, fresh oxygen containing gas is preferably added to every reactor in the series. The oxidation mixture feed and oxygen containing gas feed are preferably fed separately although conceivably these could be fed together.

The oxygen containing gas is preferably washed before use. Ideally this is achieved using first a diluted caustic solution, e.g. a solution having a pH of 8-12, preferably 10-12, in order to remove all acidic traces, such as SO₂ and CO₂. Water can then be used in order to remove any caustic traces.

Typical CHP production rate is in the range 15 to 50 kg CHP /(m³h). Preferably from 20 to 30 kg CHP / (m³h).

The oxidation reactors are preferably operated with reducing liquid levels, whereby the driving force for passing the oxidation mixture from one reactor to the next one is gravity. This minimizes the use of pumps which is economic in terms of energy consumption but also reduces the amount of heat present and hence CHP decomposition.

In the context of the present invention, the term "reducing liquid levels" means that the upper surface of the oxidation mixture gets lower in each oxidation reactor following the first one. The term is not intended to limit the way in which the reactors are positioned, although the reactors may be placed at reducing heights relative to the ground or relative to sea level. The term reducing liquid levels does not therefore mean less oxidation mixture is present, only that the upper surface of the oxidation mixture is lower relative to sea level than the previous oxidizer.

This may also be achieved by increasing the diameter of the reactor to increase volume and so on.

The reactors are all preferably operated at a pressure of 0-10 barg. According to a preferred embodiment, the oxidation is operated at a pressure above atmospheric pressure, e.g. 1.0 to 8.0 barg, preferably at a pressure of 2.0-8.0 barg, more preferably 2.5-6.0 barg, most preferably 3.0-4.5 barg. These pressures are present therefore in every reactor in the series.

It will be appreciated however that there is no requirement to operate all reactors at the same pressure. Optionally, a higher pressure may be used in the first reactor than in the following ones. Thus, the first reactor could be operated at a pressure of 4.5-5.5 barg, while the following reactors could be operated at a pressure of 3.5―4.5 barg.

The temperature generally decreases when going downstream from the first reactor. Thus, the first reactor is operated at the highest temperature and the last reactor is operated at the lowest temperature. The operating temperature is 90-115°C, preferably 95-110°C. Since the oxidation is an exothermic reaction, the reactors following the first one may require external cooling, while the first reactor may require heating to the required temperature level, which may take place for example by heating the cumene feed or by heating the circulating streams with steam.

According to a preferred embodiment, the operating temperature is 95-115°C when the number of reactors is six, whereas it is 96―110°C when the number of reactors is three.

According to the present invention, the concentration of CHP at the outlet of the last oxidation reactor is preferably 22-32%, more preferably 24-28%.

It is preferred if the cumene feed enters the bottom section of the reactors although this is by no means critical. The term "bottom section" of a reactor will be readily understood by the skilled man. This means less than halfway up (vertically) the reactor in question.

It is also preferred if the oxygen containing gas feed enters the bottom section of the reactors.

The oxidation is operated as a dry oxidation, whereby the only feeds conducted to the oxidation reactors are the cumene feed and the oxygen containing gas feed.

According to another preferred embodiment of the invention, the off-gases are separated from the oxidation mixture at the top section of each reactor. These may be treated, for example to remove unreacted cumene. The residual off gases from the oxidation reactors may then be incinerated in a regenerative thermal oxidizer (RTO).

### Concentration Step

The resulting product mixture from cumene oxidation typically contains 22-32 wt%, preferably 24-28 wt% of CHP. It may contain around 70 wt% cumene. Before the cleavage step to convert the CHP to phenol, the concentration of CHP in the reaction mixture must preferably be raised to over 70 wt%, preferably over 75 wt%, especially over 80 wt%. The ideal CHP concentration is about 82 wt%.

If the oxidation step has been performed at elevated pressure, light gases like nitrogen and air may first be flashed from reaction mixture at lower pressure in order to decrease loading on the vacuum system in the concentration stage. This is not the same as a first stage flash evaporation step as it does not separate cumene from CHP.

The concentration process of the present invention employs at least 2 steps, preferably 3 to 4 steps. Most preferably three steps are used. The use of three steps ensures the ideal heat surface area and residence time of CHP in the concentration process. Each step in the concentration process takes place within a different vessel, in particular a different distillation column.

A conventional first step in the concentration of CHP is simply a flash step. As cumene has a much higher vapour pressure and lower boiling point than CHP (cumene boiling point is 152.4°C at atmospheric pressure), it is possible to separate CHP and cumene using a flash step. Cumene can be flashed off and condensed and cleaned for recycling to the oxidation stage. CHP in the condensate is preferably 1 wt% or less.

The boiling point of CHP is about 118°C at 2 kPa. CHP decomposes significantly however at 120°C or above. Preventing CHP degradation is therefore a problem during a flash step. Also, if the amount of flashed cumene is increased then the CHP concentration increases in the condensate. This leads to CHP losses. Therefore, the use of a flash step is not without disadvantage.

The present inventors have realised that maximizing the load in the first step is valuable to ensuring a successful overall concentration process. In order to achieve this, the inventors propose moving away from a flash step. It is preferred therefore if the first step in the process of the invention involves a distillation column, in particular one in which random or structured packing is involved. By employing a distillation column rather than a simple flash step, the load on the first step can be increased. It is preferred therefore if the load in the first step is higher than that of the subsequent step(s). Alternatively, it is preferred if the load in the first two steps is higher than that of a third step of the concentration process.

The term concentration column and distillation column are used interchangeably herein. The term tower may also be used to substitute the term column.

By higher load it is meant that a concentration step, e.g. the first step or perhaps the first two steps, have a greater reboiling duty and a larger amount of cumene is separated in first or second distillation column.

As the CHP concentration in the first stage is at its lowest, the temperature within the first step of the concentration process can be at its highest as there is the lowest risk of inducing degradation of the CHP.

It is important that the temperature during the concentration stages is kept below 110°C, e.g. 90 to 105°C, preferably 100°C or less. If a temperature above this is employed, there is a much higher risk of degradation of CHP. The temperatures in any column of the concentration process is preferably 100°C or less, such as 90 to 100°C.

The present inventors have also realized that minimizing the temperature in the later stages of the process is vital. As the concentration process proceeds the content of the CHP in the reaction mixture increases. This makes the reaction mixture more susceptible to degradation. It is a preferred feature of the invention therefore that the temperature should decrease from one step to the next during the concentration process. The temperature within the columns preferably decreases by at least 1°C from the first to the second column. It is also preferred that where there are 3 or more columns, the temperature decreases from the second to the third column and so on.

The distillation columns used in the concentration process of the invention preferably contain random or structured packing. The cumene which is distributed at the top of the packing can be distributed evenly using low pressure drop liquid distributors as is known in the art. Structured column packing is formed from vertical sheets of corrugated thin gauge ceramic, plastic or metal with the angle of the corrugations reversed in adjacent sheets to form a very open honeycomb structure with inclined flow channels and a relatively high surface area.

The use of a high surface area materials increases vapour liquid-contact. The low resistance to vapour flow together with efficient use of available surface tends to give structured packing performance advantages over random packing especially in high vapour rate/low liquid rate systems.

Corrugated structured packing/structured column packing is preferably constructed of sheets of corrugated metal. The angle of inclination of the corrugations of adjacent sheets is reversed with respect to the vertical column axis, forming mixing cells at every point where the corrugations intersect. Perforations and surface texturing, including waffled, grooved and smooth surfaces, promote intimate mixing and radial distribution over the entire column/tower cross-section. Each subsequent element can be rotated by 90°C so that the sheets of one element are perpendicular to the sheets of the elements above and below.

In passing through each element, gas and liquid are thoroughly mixed in the direction parallel to the plane of the sheets. By rotating subsequent elements, excellent mixing and spreading, both side-to-side and front-to-back, are obtained over the entire cross-section of the tower.

An alternative is to use ceramic corrugated structured column packing. This may be required where plastic or metal sheets would be damaged by reactants/conditions present during the reaction.

Structured packing improves hydraulic performance as the corrugated sheets are vertically oriented, eliminating any horizontal surfaces which create resistance to fluid flow. As a result, towers packed with corrugated structured column packing have a very low pressure drop and a corresponding higher capacity.

A further option is gauze structured column packing. Gauze is made from a woven metal wire or plastic mesh and is ideal for applications where low pressure drop is important and liquid rates are low or temperature-sensitive compounds are present. Typical gauzes might have a surface area of 500 m² or higher, e.g. 750 m².

A cheaper alternative to the use of structured packing is the use of random packing. Both these alternatives offer better results than the use of trays alone in the distillation tower. Random packing is typically formed from small ceramic, plastic or metal obj ects with high surface area which are packed together in the column to massively increase surface area within the column.

Typical random packing objects are based on saddle rings, cascade rings, pall rings, cross-partition rings, Y form rings, conjugate ring, raschig ring, oval hole chain, Y chain, ceramic ball, saddles and so on. Random packing provides better drain of CHP from concentration stages in case of unplanned shut down of the concentration section.

Both structured and random packing solutions are available for commercial purchase. Conventional reaction set ups currently employ sieve trays (often four sieve trays) in the distillation columns of the second and third concentration stages. The change to structured or random packing improves separation efficiency and enables shorter liquid residence times. This in turn reduces impurities and maximises yields. Random packing is preferably used for its relative economy but still excellent benefits. In particular, the presence of packing reduces pressure drop during the concentration process and allows for decreased pressures and temperatures to be employed in reboilers.

It is possible to use random or structured packing in one or more of the concentration columns. It is particularly preferred to use random or structured packing in the last two concentration columns in the series, e.g. columns 2 and 3 where there are three columns present. It is most preferred to use random or structured packing in all columns.

Moreover, it is generally preferred if the pressure within the concentration columns decreases from the first step to the last step. For example, the pressure within the first step may be between 10 to 20 kPa, reducing by 3 to 8 kPa to the second step and reducing again in any subsequent step. We have surprisingly observed however that the pressure needed in the present invention may be higher in the first step than is required in conventional operation.

It is beneficial to run the first step at higher pressure. This allows smaller volumetric flows and less concentrator piping and other auxiliary equipment is smaller.

It is beneficial for the invention that the residense time in columns is minimized by column and piping and reboiler design.

During the concentration process cumene is removed from each concentration column via an overhead stream. It is conventional in the art to recycle cumene removed during the concentration step as this is a valuable resource. The cumene is typically washed and recycled back to the start of the oxidation reaction where it can be converted to CHP. The cumene removed is preferably subjected to purification procedures discussed below.

The present inventors have surprisingly realised however, that the concentration process can be improved if fresh cumene is fed directly to at least the first of the concentration steps, preferably to at least the first and second concentration steps, especially to all the concentration steps. Whilst it is well known to recycle cumene back to the oxidation step, it is believed that no one before has suggested adding a fresh cumene feed directly to the first step of the concentration process. Given that cumene is the compound that the skilled man is actually trying to remove from the CHP, it is counter intuitive to consider adding it directly to the concentration process. However, we have found that its use is valuable.

Fresh cumene is preferably fed to the concentration column via its own dedicated conduit. It is preferred if cumene is fed to a concentrator at the top of the column, i.e. more than 50 % of the way up the column, preferably more than 75% of the way up. Ideally, fresh cumene is fed above the top of the packing present within the column.

The term fresh cumene refers to cumene taken from an essentially pure cumene source, e.g. 95 wt% cumene, preferably 99 wt% cumene or more. The term is used herein to distinguish cumene that might be returned to a concentrator via reflux. A conventional means to arrange reflux would be to return part of the condensate to the column. That is not a fresh cumene feed herein. The condensate contains impurities like organic acids which are likewise recycled to the column. By using the fresh cumene addition according to the invention this can be avoided, which is a significant advantage.

It is also beneficial to have a low CHP concentration in condensates which are treated in caustic wash because CHP reacts with NaOH to give sodium peroxide and therefore CHP loss increases. By using the fresh cumene stream according to the invention the CHP concentration in the condensate is minimized.

We reiterate that the fresh cumene feed here is separate from any cumene inherently present within the oxidation mixture and hence fed to the concentration reactors via the oxidation step. The cumene added is a separate cumene feed, based on essentially pure cumene.

This cumene feed must therefore be fresh cumene, i.e. cumene which is purified and essentially free of most impurities such as carbonates and other salts. Typically therefore this can derive from the feed to the oxidation reactors.

It is preferred therefore that instead of all the fresh cumene being sent to the oxidation step(s), cumene can be split and part sent to the oxidation step and part sent direct to the concentrator process.

The amount of fresh cumene sent to the concentration process can vary. Preferably, 2 to 10 wt%, such as 4 to 7 wt% of feed to the first or second concentration column is fresh cumene. In the last step of concentration, cumene may form 15 to 30 wt%, such as 22 to 28 wt% of the total feed to that step. Because of the higher CHP concentration in the feed of the last step, a higher fresh cumene amount is required in order to minimize CHP losses from the top of the column.

It will be appreciated that the fresh cumene feed may be added as reflux.

All the fresh cumene can be feed to the first concentration column but it is preferred if some fresh cumene is sent to each concentration column. The fresh cumene is preferably fed as a liquid.

It is beneficial to provide concentration columns with fresh cumene as opposed to condensate reflux because the impurities are not recycled back to concentration section. The degradation of CHP decreases due to smaller amount of impurities like organic acids and less impurities end up to concentrated CHP product.

It is also beneficial to minimize CHP concentration in condensates which are treated in caustic wash. CHP reacts with NaOH to form sodium peroxide and CHP loss increases.

It is preferred if each concentration column in the series is positioned in such a way that gravity can be used to transfer material from an earlier to a later column. This is obviously cheaper and avoids the use of expensive pumps. Moreover, by using gravity we avoid the risk of CHP decomposition within pumps. This can be achieved most readily by setting columns at lowering heights relative to each other.

It will be appreciated that reboilers can be used as is well known in the art to drive distillation. Reboilers may be used to boil the liquid from the bottom of a previous distillation column to generate vapors which are returned to the bottom of the next distillation column to drive the distillation process column. Reboilers will generally be used therefore between oxidation and concentration steps and between each individual concentration step. It will be appreciated that any cumene transferred between concentration columns via this mechanism is not a fresh cumene feed.

The first step reboiler may however be a conventional heat exchanger for evaporating the oxidate mixture feed to the first concentration column. This is very beneficial because conventional heat exchanger can be designed for a very short residence time compared to conventional reboiler.

The cumene removed overhead during the concentration step has to be cleaned before it is returned to the process of the invention. The use of a caustic wash followed by water wash is a preferred method for purifying the cumene. The caustic wash does however generate carbonates and sodium phenate which need to be removed. The use of a subsequent water wash generally removes all carbonates as these partition into the water layer. Sodium phenate also partitions into water. By eliminating carbonates and other salts during this washing step these are not fed back to the cumene source and hence the cumene source is essentially pure. The presence of caustic residues can damage heat exchanger surfaces (reboilers) and can encourage CHP degradation. By eliminating these we ensure clean reboilers and lower CHP degradation.

It is preferred if a stream from the water wash step is returned to the caustic wash step and at least part of the aqueous stream from the caustic wash step is purged. This avoids build up of any impurities or unwanted compounds within the washing procedure. It is also within the scope of the application for their to be a partial recycle at the caustic step and a partial recycle at the water step.

The CHP concentration in the reaction mixture after concentration is preferably at least 70 wt%, more preferably at least 75 wt%, such as at least 80 wt%. It is preferred if the impurities present after concentration are present at very low levels. The content of acetophenone may be less than 0,6%. The content of dimethylphenolcarbinol may be less than 4%.

After the CHP has been concentrated by the process of the invention, the cleavage of the CHP to phenol can be carried out by conventional means. A discussion of the necessary techniques is not required here.

### Brief Description of the Figures

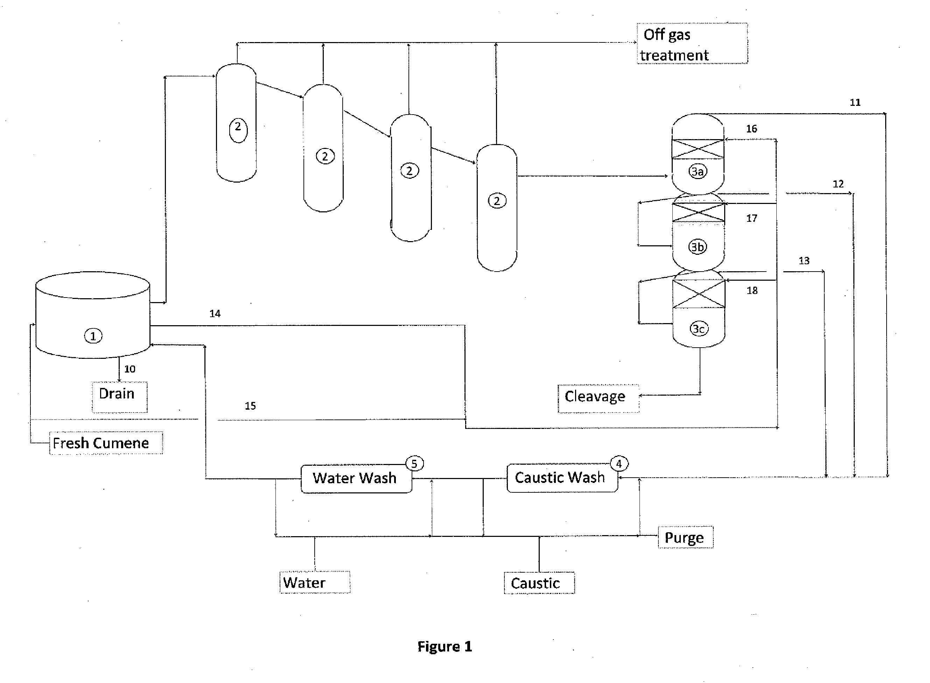

Figure 1 is a drawing of the first two stages of a cumene oxidation process in which cumene is converted by oxidation to CHP and the CHP concentrated to a sufficient level to be employed in the subsequent cleavage process.

Cumene tank (1) is supplied with fresh cumene from a suitable source. It is also provided with water drain (10) to remove any water carried over from the water wash vessel (5).

Cumene is fed from tank (1) to the first in a series of oxidation reactors (2). The oxidation reactors (2) are positioned at sequentially decreasing heights relative to each other so that transfer of material between them can be accomplished simply using gravity. Off gases are taken from each reactor (2) and treated. Any cumene within those off gases is typically recovered and reused (not shown). Residual off gases are preferably removed for incineration in a thermal oxidizer. An oxygen containing gas is preferably supplied to each oxidation reactor (2) (not shown).

The reaction mixture from the final oxidation reactor (2) is passed to first concentrator (3a) operated under cumene reflux. The first concentrator is formed of a column/tower containing random or structured packing.

Material from concentrator (3a) passes to concentrator (3b) and then on to concentrator (3c). Both concentrators (3b) and (3c) are preferably distillation columns/towers and contain liquid distributors (not shown) and random or structured packaging. It is preferred if all the columns contain structured packing or random packing. The concentrated reaction mixture from concentrator (3c) is transferred to cleavage to phenol and acetone (not shown).

Transport between concentrators can be accomplished simply using gravity as these can be arranged at sequentially decreasing heights relative to each other. The avoidance of pumps to transport material is preferred.

Cumene is removed overhead from each of concentrators (3a) to (3c) via conduits (11-13). It will be appreciated that transfer lines between the oxidation step and the concentration step and lines between the concentration steps themselves can be provided with reboilers as is known in the art. These may boil the liquid from the bottom of a distillation column to generate vapors which drive the distillation separation.

The cumene removed overhead in lines (11 to 13) is condensed and combined and washed in caustic in washer (4) and subsequently water washed in washer (5). Caustic and water can be recycled and/or purged. Ideally, a stream from the water wash step (5) is recycled back to the caustic wash step (4) and a stream from the caustic wash step (4) is purged to avoid build up of any impurities or unwanted compounds. The washed cumene then returns to tank (1).

Conduit 14 transfers cumene from tank (1), to concentrators (3a), (3b) and (3c). Fresh cumene may alternatively be supplied directly through line 15 and divided to concentrators (3a-c) through lines 16, 17 and 18.

The invention will now be described with reference to the following non limiting examples.

### Examples

The benefits of the present invention are demonstrated by presenting two examples. The arrangement of concentration columns in example 2 is according to the figure 1 and the invention. Heat exchanger arrangements are not shown in the figure. Each concentrator has reboiler/condenser as known in the art.

Example 1 is based on the first concentration step being a simple flash without cumene or condensate reflux.

### Example 1:

● CHP Concentration steps consist of a first flash vessel, concentration column 3b and concentration column 3c;
● Concentration columns have trays;
● Columns have 4 trays.

### Example 2: Case 1 has been modified according to present invention:

● Concentration steps : concentration column 3a Concentration column 3b concentration column 3c;
● Trays are replaced by random packing in concentration columns 3b and 3c;
● Feed flash vessel has been replaced by a Concentration column 3a;
● Concentration column 3a has random packing;
● Fresh cumene feed to the top of all concentration columns;
● Fresh cumene to column 3a is 4 wt% of feed to the 3a;
● Fresh cumene to column 3b is 6 wt% of feed to the 3b;
● Fresh cumene to column 3c is 20 wt% of feed to the 3c.
● Bottom temperature in different steps is about 100 to 104°C.

### Other features:

● In both cases CHP concentration in combined condensate is about 1 wt%.
● Diameters of concentration column 3b and 3c are same size in both cases;

In table 1 relative differences are presented:

**Table 1**

| | Example 1 | Example 2 |
|---|---|---|
| Relative Feed | 1 | 2.8* |
| Feed CHP to 1 st column | 22 wt% | 27 wt% |
| Bottom CHP (last column) | | 82 wt% |

| | | |
|---|---|---|
| *Capacity increase because of higher cumene evaporation of step A in the concentration stage compared to example 1 | | |

**Table 2: Percentage of total cumene evaporated of total feed to step (includes reflux)**

| | Example 1 | Example 2 |
|---|---|---|
| Step flash/3a | 28,5 w-% | 40.0 w-% |
| Step 3b | | 31.3 w-% |
| Step 3c | | 28.4 w-% |

**Table 3 : Operation pressure**

| | Example 1 | Example 2 |
|---|---|---|
| Step flash/3a | 18 kPa | 16 kPa |
| Step 3b | | 11 kPa |
| Step 3c | | 4 kPa |

By replacing the flash step by a concentration column and using random packing and using fresh cumene reflux it is possible to load the first step significantly. This example also demonstrates that Columns A and B can be even same size although the capacity has increased about 2.8 times.

## Claims

1. A process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least the first of said concentration columns is provided with a fresh cumene feed.

2. A process for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least one of said concentration columns comprises random or structured packing.

3. A process as claimed in claim 1 or 2 for the concentration of cumene hydroperoxide comprising:
(I) transferring the reaction mixture from the oxidation of cumene to cumene hydroperoxide to the first of at least two concentration columns arranged in series;
(II) distilling the reaction mixture in said concentration columns to increase the percentage of cumene hydroperoxide within the reaction mixture and removing cumene from the reaction mixture in each concentration column via an overhead stream;
wherein at least one of said concentration columns comprises random or structured packing; and
wherein at least the first of said concentration columns is provided with a fresh cumene feed.

4. A process for the conversion of cumene to cumene hydroperoxide and the subsequent concentration thereof as claimed in claim 1 to 3 comprising:
(I) oxidising cumene in the presence of an oxygen containing gas in at least one oxidation reactor, preferably at least 3 oxidation reactors connected in series, so as to form a reaction mixture containing cumene and cumene hydroperoxide;
(II) transferring the reaction mixture of stage (I) to the first of at least two concentration columns arranged in series;
(III) distilling the reaction mixture in said concentration columns to increase the percentage of cumene hydroperoxide within the reaction mixture and removing cumene from the reaction mixture in each concentration column via an overhead stream;
wherein at least the first of said concentration columns is provided with a fresh cumene; and/or
wherein at least one of said concentration columns comprises random or structured packing.

5. A process as claimed in any preceding claim wherein all columns are provided with a fresh cumene feed.

6. A process as claimed in any preceding claim wherein all columns are provided with random or structured packing.

7. A process as claimed in any preceding claim wherein there are three concentration columns.

8. A process as claimed in any preceding claim wherein there is a higher load in first or first and second columns relative to any later columns.

9. A process as claimed in any preceding claim wherein all columns comprise random packing.

10. A process as claimed in any preceding claim wherein the concentration step is carried out at a temperature below 110°C.

11. A process as claimed in any preceding claim wherein cumene is fed to the top of said at least one concentration column.

12. A process as claimed in any preceding claim wherein transfer between concentration columns is effected under gravity.

13. An apparatus for use in a process as hereinbefore defined comprising:
a cumene source (1) arranged to provide cumene to a first oxidation reactor (2), said first oxidation reactor being the first in a series of at least three oxidation reactors connected in series;
at least two concentration columns (3A-3C) connected in series arranged to receive reaction mixture from the last in said series of oxidation reactors;
said concentration columns each having an overhead conduit arranged to remove cumene from said columns for recycling via a caustic (4) and then water (5) washing means to said cumene source (1);
said cumene source being arranged to provide fresh cumene via a conduit (14)/(15) to at least first of said concentration columns.

14. An apparatus as claimed in claim 13 wherein one of more of said concentration columns comprises random or structured packing.

15. An apparatus as claimed in claim 14 wherein said concentration columns each having a liquid distributor to distribute fresh cumene on the packing.
